# EUROPÄISCHE PATENTSCHRIFT

(11) **EP 3 917 441 B1**
(45) Veröffentlichungstag und Bekanntmachung des Hinweises auf die Patenterteilung: **04.06.2025**
(21) Anmeldenummer: 20701009.1
(22) Anmeldetag: 15.01.2020
(51) Int. Cl.: A61B 90/70, A61B 17/16, A61B 90/50

(54) **HALTEVORRICHTUNG FÜR CHIRURGISCHE HANDSTÜCKE**
HOLDING DEVICE FOR SURGICAL HANDPIECES
DISPOSITIF DE MAINTIEN POUR PIÈCES À MAIN CHIRURGICALE

(30) Priorität: 29.01.2019 DE 102019102194
(43) Veröffentlichungstag der Anmeldung: 08.12.2021
(73) Patentinhaber: Aesculap AG, 78532 Tuttlingen (DE)
(72) Erfinder: HÖGERLE, Roland-Alois, 78532 Tuttlingen (DE); PFISTER, Ralf, 78647 Trossingen (DE); MILLER, Simon, 78652 Dreißlingen (DE); BÜRK, André, 78056 Villingen-Schwenningen (DE); MACHILL, Martin, 78604 Rietheim-Weilheim (DE); BLUST, Edgar, 78126 Königsfeld (DE); ROTH, Manuel, 78166 Donaueschingen (DE); AUBER, Stephanie, 78532 Tuttlingen (DE)
(74) Vertreter: Winter, Brandl - Partnerschaft mbB
(86) Internationale Anmeldenummer: PCT/EP2020/050931
(87) Internationale Veröffentlichungsnummer: WO 2020/156814

(56) Entgegenhaltungen:
- DE-A1- 4 323 815
- US-A- 2 878 895
- US-A- 3 556 669
- US-A1- 2003 000 774
- US-A1- 2005 236 230

## Beschreibung

Die vorliegende Offenbarung betrifft eine Haltevorrichtung zum Halten und Lagern von zumindest einem medizinischen Instrument, insbesondere einem chirurgischen Handstück, mit einer zentralen Zuleitung, zumindest einem mit einem Ende der zentralen Zuleitung verbundenen Leitungsabschnitt, an welchem das zumindest eine medizinische Instrument angeordnet ist, und einem an einem anderen Ende der zentralen Zuleitung ausgebildeten Anschlussabschnitt.

### Hintergrund der Erfindung

Zur Aufbereitung und Reinigung chirurgischer Handstücke werden üblicherweise Haltevorrichtungen verwendet. Bei den chirurgischen Handstücken handelt es sich beispielsweise um Fräser oder Shaverhandstücke, die einen Längskanal aufweisen, welcher manuell mit Bürsten nicht reinigbar ist, da durch Borsten Verschmutzungen im Inneren der Handstücke zwar möglicherweise gelöst werden, jedoch nicht vollständig entfernt werden können und somit Verschmutzungsrückstände zurückbleiben können.

Der vollständig Aufbereitungs- und Reinigungsprozess der chirurgischen Handstücke gliedert sich dabei in eine manuelle Vorreinigung, eine maschinelle Reinigung und Desinfektion, einen Pflegeschritt und einen Sterilisationsschritt. Zur manuellen Vorreinigung wird das Handstück auf einer Haltevorrichtung platziert. Im Anschluss daran wird eine Wasserpistole an einen Anschluss der Haltevorrichtung für Wasser- und Druckluftpistolen angebracht und das Handstück mit Wasser durchspült. Der Anschluss für den Spülschlauch (die Wasserpistole) muss dabei mit Zellstoff oder einem ähnlichen Material abgedeckt werden, damit ein ungewolltes Ausströmen der Reinigungsflüssigkeit verhindert wird. Für die maschinelle Reinigung und Desinfektion werden die Handstücke auf eine in einem bekannten Reinigungs- und Desinfektionsgerät platzierte Haltevorrichtung gesteckt und maschinell mit einer Reinigungsflüssigkeit und einer Desinfektionsflüssigkeit umspült. Nach der maschinellen Reinigung und Desinfektion werden die Restflüssigkeiten mit Hilfe der Haltevorrichtung und der Druckluftpistole durch manuelles Ausblasen aus dem Handstück entfernt. Nachdem das Handstück auf eine Umgebungstemperatur abgekühlt ist, wird der Pflegeschritt mit einem Ölspray durchgeführt. Üblicherweise müssen dafür die Handstücke entnommen, einzeln auf einen Ölspray-Adapter gesetzt und für eine gewisse Zeit, vorzugsweise einige Sekunden, mit dem Ölspray durchsprüht werden. Zur Sterilisation wiederum lagern die Handstücke auf einem Siebkorb, da das in der Haltevorrichtung vorgesehene Filtermaterial nicht für eine Dampfsterilisation, wie sie üblicherweise bei der Sterilisation chirurgischer Handstücke zum Einsatz kommt, vorgesehen ist.

### Stand der Technik

Mit herkömmlichen Haltevorrichtungen kann es möglich sein, zumindest die Reinigungs- und Pflegeschritte ohne aufwändiges manuelles Umstecken der Handstücke durchzuführen. In der DE 10 2010 017 624 A1 ist beispielsweise eine Haltevorrichtung zum Reinigen und Lagern chirurgischer Handstücke und ein Siebkorb mit der Haltevorrichtung offenbart. Die Haltevorrichtung weist dabei einen Reinigungsfluidanschluss zum Einleiten eines Reinigungsfluids und einen Verteiler zwischen dem Fluidanschluss und mindestens zwei Handstückaufnahmen, auf welche jeweils ein Handstück gesteckt werden kann, auf. Zwischen dem Reinigungsfluidanschluss und den Handstückaufnahmen ist eine Filtereinrichtung zur Filterung des Reinigungsfluids angeordnet, jedoch ist diese Filtereinrichtung nicht für die Dampfsterilisation vorgesehen, so dass die Haltevorrichtung lediglich für die manuelle und maschinelle Reinigung und Desinfektion verwendbar ist.

Eine weitere Haltevorrichtung mit der Möglichkeit, die Reinigungs- und Pflegeschritte ohne manuelles Umstecken durchzuführen, ist beispielsweise in der US 3 811 208 A offenbart. Die pneumatische Reinigungs-, Desinfektions- und Schmiervorrichtung (Ölvorrichtung) für ein rohrförmiges dentales Handstück umfasst dabei ein kompaktes und tragbares Reservoir, in welchem je nach Anwendung eine Reinigungs-, Desinfektions- oder Öllösung gespeichert ist, und eine Druckluftquelle, über welche der Vorrichtung Druckluft zugeführt wird, so dass die Lösung in dem Reservoir sukzessive und zwangsweise in einen zerstäubten Zustand überführt wird. Die zerstäubte Flüssigkeit wird im Anschluss durch das aufgesteckte dentale Handstück geleitet. Eine derartige Vorrichtung ist jedoch nicht zur Sterilisation und Lagerung vorgesehen und dient nur der Reinigung und Pflege der Handstücke. Des Weiteren können die Handstücke hierbei nur einzeln durchsprüht werden.

Die US 2005/236230 A1 offenbart ebenfalls einen beispielhaften Instrumentenanschluss und einen Reinigungseinheitsanschluss mit einem Ölvorratsbehältnis.

### Kurzbeschreibung der Erfindung

Ausgehend von dem bekannten Stand der Technik zeigt sich der Nachteil, dass während des gesamten Aufbereitungs- und Reinigungskreislaufs die Handstücke in verschiedenen Lagerungsvorrichtungen, insbesondere ein Siebkorb zur Sterilisation und Lagerung, eine Haltevorrichtung zur manuellen und maschinellen Reinigung und Desinfektion und ein Ölspray-Adapter zur Pflege und Ölung, gelagert werden müssen. Die Tatsache, dass die Handstücke mehrmals händisch entnommen und zwischen den verschiedenen Lagerungsvorrichtungen umgesteckt werden müssen, erhöht die Gefahr einer Beschädigung oder einer Kontamination/Verschmutzung der Handstücke.

Es ist daher die Aufgabe der Erfindung, die Nachteile aus dem Stand der Technik zu vermeiden oder wenigstens zu mildern. Insbesondere soll eine Haltevorrichtung für chirurgische Handstücke bereitgestellt werden, welche die Reinigung und Desinfektion, die Pflege, die Sterilisation und die Lagerung der Handstücke während des gesamten Aufbereitungskreislaufs mit nur einer Haltevorrichtung, ohne manuelles Umstecken der Handstücke zwischen den einzelnen Aufbereitungsschritten bzw. mit einer möglichst geringen Anzahl an (manuellen) Aufbereitungsschritten, ermöglicht.

Diese Aufgabe wird durch eine gattungsgemäße Vorrichtung mit den Merkmalen des Anspruchs 1 gelöst. Insbesondere wird die Aufgabe bei einer gattungsgemäßen Vorrichtung nach einem ersten erfindungsgemäßen Aspekt dadurch gelöst, dass der Anschlussabschnitt der erfindungsgemäßen Haltevorrichtung für medizinische Instrumente dafür ausgebildet ist, mit einer Vorreinigungseinheit, insbesondere einer Wasser-/Druckluftpistole, und einer Reinigungseinheit, insbesondere eine mit einer Spülleitung verbundenen Filtereinheit, wahlweise gekoppelt zu werden und der andere Endabschnitt der zentralen Zuleitung, d.h. der dem Anschlussabschnitt naheliegende Endabschnitt einer in eine Anzahl von medizinischen Instrumente haltenden Zweigleitungen sich auffächernde zentralen Zuleitung, in der Art einer Venturi-Düse ausgestaltet ist, an deren Stelle mit relativ kleinem Querschnitt abzweigend ein Ölanschluss als ein an einem Ölreservoir koppelbarer Stechdorn ausgebildet ist.

Dies hat den Vorteil, dass die Haltevorrichtung das medizinische Instrument, insbesondere ein chirurgisches Handstück, während des gesamten Aufbereitungszyklus hält und somit die Gefahr einer Kontamination oder Verschmutzung nach Reinigung und Pflege gemindert wird. Wenn nun vorzugsweise an dem, dem Anschlussabschnitt naheliegenden Endabschnitt der zentralen Zuleitung ein mit einer Öleinheit, insbesondere einem Einweg-Ölvorratsbehältnis, koppelbarer Stechdorn angeordnet ist, führt die Venturi-Düse dazu, dass sich bei Durchströmung der Venturi-Düse, d.h. bei Durchströmung des Endabschnitts, ein Unterdruck in dem Endabschnitt ausbildet, aufgrund welchen Öl aus der Öleinheit gesaugt wird. Dies hat wiederum den Vorteil, dass auch bei der Ölung, welche Teil der Pflege darstellt, das medizinische Instrument bzw. die Haltevorrichtung nicht manuell operiert werden müssen bzw. die Anzahl der einzelnen Handhabungsschritte während des gesamten Aufbereitungsprozesses reduziert werden kann. Zudem entfällt durch diese Ausgestaltung vor der Pflege bzw. Ölung der Haltevorrichtung und des zumindest einem chirurgischen Handstücks das manuelle Ausblasen von Spülflüssigkeitsresten.

Ferner ist in dem zumindest einen Leitungsabschnitt der Haltevorrichtung zwischen Anschlussabschnitt und gehaltenem medizinischen Instrument eine Ventileinrichtung angeordnet, welche in einer Grundstellung, in welcher der Leitungsabschnitt und das chirurgische Handstück entkoppelt sind, geschlossen ist und sich bei Kopplung des chirurgischen Handstücks an den Leitungsabschnitt öffnet. Wenn nun beispielsweise das chirurgische Handstück ohne Kenntnis fehlerhaft angekoppelt ist, wird die Gefahr, dass beispielsweise bei der Reinigung zugeführtes Spülwasser an dem fehlerhaft gekoppelten Leitungsabschnitt austritt, gegenüber dem bekannten Stand der Technik verringert.

Vorteilhafte Ausgestaltungen sind in den Unteransprüchen beansprucht und werden nachstehend näher erläutert.

Vorzugsweise kann der Anschlussabschnitt als Luer-Lock-Verbindung ausgestaltet sein.

In einer weiteren bevorzugten Ausgestaltung kann der Anschlussabschnitt dafür vorgesehen sein, um mit einer Öleinheit, insbesondere einem Ölspray oder einem Ölreservoir, koppelbar zu sein. Das zumindest eine chirurgische Handstück muss daher zur Pflege bzw. Ölung nicht von der Haltevorrichtung abgekoppelt werden, so dass sowohl das zumindest eine chirurgische Handstück als auch Funktionsteile der Haltevorrichtung gleichzeitig in einem Schritt geölt werden können.

Des Weiteren betrifft die Erfindung ein Aufbereitungs- und Reinigungssystem mit einer offenbarungsgemäßen Haltevorrichtung, einer Vorreinigungseinheit, welche dafür ausgebildet ist, an den Anschlussabschnitt der Haltevorrichtung angeschlossen zu werden, und einem Ölreservoir, das dafür ausgebildet ist, an den Ölanschluss der Haltevorrichtung angeschlossen zu werden.

### Kurzbeschreibung der Figuren

Fig. 1 ist eine schematische Darstellung einer Haltevorrichtung zum Halten und Lagern von chirurgischen Handstücken gemäß einem nicht erfindungsgemäßen ersten Ausführungsbeispiel; und
Fig. 2 ist eine schematische Darstellung einer Haltevorrichtung zum Halten und Lagern von chirurgischen Handstücken gemäß der Erfindung.

### Beschreibung der Ausführungsbeispiele

Nachstehend wird ein bevorzugtes Ausführungsbeispiel der vorliegenden Offenbarung auf der Basis der zugehörigen Figuren beschrieben.

### Bevorzugtes Ausführungsbeispiel

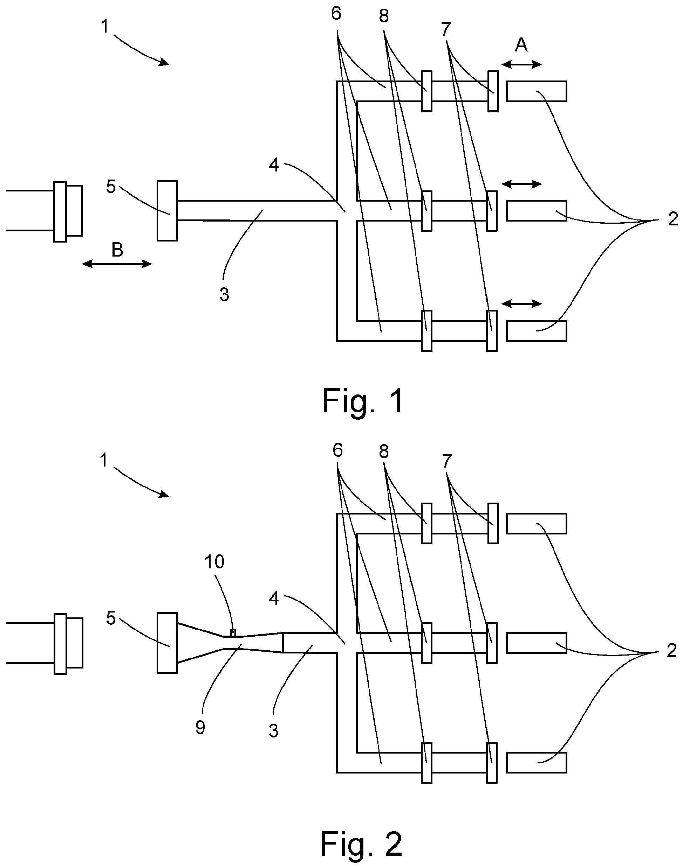

Fig. 1 ist eine schematische Darstellung einer Haltevorrichtung 1 zum Halten und Lagern von chirurgischen Handstücken 2 während eines Aufbereitungsprozesses der chirurgischen Handstücke 2. Die Haltevorrichtung 1 umfasst in dem bevorzugten Ausführungsbeispiel eine zentrale Zuleitung 3, welche an einem ersten Endabschnitt mit einem Verteilabschnitt 4 in Fluidverbindung steht und an einem zweiten Endabschnitt einen Anschlussabschnitt bzw. eine Leitungskupplung 5 aufweist.

In dem bevorzugten Ausführungsbeispiel teilt sich die zentrale Zuleitung 3 an dem Verteilabschnitt 4 in vorzugsweise drei Leitungsabschnitte/Zweigleitungsabschnitte 6 auf, an deren dem Verteilabschnitt 4 abgewandten Endabschnitten/Enden die chirurgischen Handstücke 2 jeweils über eine Koppeleinrichtung 7, wie in Fig. 1 durch die Bewegungspfeile A angedeutet, koppelbar angeordnet sind, so dass im gekoppelten Zustand der Anschlussabschnitt 5 der Haltevorrichtung 1 über die zentrale Zuleitung 3, den Verteilabschnitt 4 und den jeweiligen Leitungsabschnitt 6 mit den chirurgischen Handstücken 2 in Fluidverbindung steht.

Des Weiteren ist in dem bevorzugten Ausführungsbeispiel an/in jedem der (drei) Leitungsabschnitte 6 jeweils eine Ventileinrichtung 8 angeordnet, welche in einem geschlossenen Zustand die vorstehend beschriebene Fluidverbindung zwischen dem Anschlussabschnitt 5 und dem jeweiligen Handstück 2 trennt und in einem geöffneten Zustand einen Fluidstrom von dem Anschlussabschnitt 5 zu dem jeweiligen Handstück 2 erlaubt. Die Ventileinrichtung 8 ist dabei so eingerichtet, dass sich die Ventileinrichtung 8 in dem geöffneten Zustand befindet, wenn an der jeweiligen Koppeleinrichtung 7 das chirurgische Handstück 2 angekoppelt ist, und sich in dem geschlossenen Zustand befindet, wenn an der jeweiligen Koppeleinrichtung 7 kein Handstück 2 angekoppelt ist.

Der Anschlussabschnitt 5 ist gemäß der vorliegenden Offenbarung (quasi als Universalanschluss) eingerichtet, um mit unterschiedlichen Einheiten, z.B. einer Filtereinheit, einer Druckluft-/Wasserpistole und einem Ölspray oder einem Ölreservoir, wie in Fig. 1 durch den Bewegungspfeil B angedeutet, koppelbar zu sein. In dem bevorzugten Ausführungsbeispiel ist der Anschlussabschnitt 5 als eine sogenannte Luer-Lock-Verbindung ausgestaltet, welche kraftschlüssig die Fluidverbindung zwischen der angekoppelten Einheit und dem Anschlussabschnitt 5 herstellt. Die jeweiligen angekoppelten Einheiten sowie deren Funktion und Zusammenwirken mit der Haltevorrichtung 1 wird nachfolgend bei der Beschreibung eines beispielhaften Aufbereitungsprozesses der chirurgischen Handstücke 2 deutlich.

### Beschreibung eines beispielhaften Aufbereitungsprozesses

Zur Aufbereitung der chirurgischen Handstücke 2 werden diese zunächst über die Koppeleinrichtungen 7 an die Haltevorrichtung 1 (mechanisch und fluidtechnisch) gekoppelt. In dem bevorzugten Ausführungsbeispiel können dabei, wie vorstehend beschrieben, bis zu drei Handstücke 2 gleichzeitig angekoppelt und aufbereitet werden, wobei auch mehr Zweigleitungen vorgesehen sein können. Als Nächstes kann optional an dem Anschlussabschnitt 5 die Wasserpistole angeschlossen werden, so dass durch Einspritzen von Wasser eine erste manuelle Vorreinigung der Haltevorrichtung 1 und der chirurgischen Handstücke 2 durchgeführt werden kann.

Im Anschluss daran wird für eine maschinelle Reinigung und Desinfektion eine Spülleitung eines Reinigungs- und Desinfektionsgeräts, über welche der Haltevorrichtung 1 eine Spülflüssigkeit zugeführt wird, an den Anschlussabschnitt 5 angekoppelt. Ein Anschlussendabschnitt der Spülleitung weist dabei die vorstehend beschriebene Filtereinheit auf, welche bei der maschinellen Reinigung der Haltevorrichtung 1 und der chirurgischen Handstücke 2 einen Eintritt von Verschmutzungspartikeln in die Haltevorrichtung 1 mit der Spülflüssigkeit verhindert. Nach dem Durchspülen der Haltevorrichtung 1 und der Handstücke 2 mit der Spülflüssigkeit wird über die Spülleitung eine Desinfektionsflüssigkeit zugeführt.

Möglicherweise in der Haltevorrichtung 1 und in den chirurgischen Handstücken 2 zurückbleibende Flüssigkeitsreste müssen vor der Ölung ausgeblasen werden. Dafür wird die Haltevorrichtung 1 von dem Reinigungs- und Desinfektionsgerät entkoppelt und an dem Anschlussabschnitt 5 wird die Druckluftpistole angekoppelt, so dass mit der Druckluftpistole eingeblasene Luft die Flüssigkeitsreste abführen kann.

Nachdem die chirurgischen Handstücke 2 auf eine Umgebungstemperatur abgekühlt sind, wird zur Pflege bzw. Ölung der Handstücke 2 und der Haltevorrichtung 1 in einem Behälter befindliches Öl zerstäubt und der Haltevorrichtung 1 und den Handstücken 2 zugeführt. Analog wie bei der für die manuelle Vorreinigung verwendeten Filtereinheit wird die Druckluftpistole an den Anschlussabschnitt 5 angekoppelt. Ein distales Ende der mit der Druckluftpistole verbundenen Leitung ist dabei nun mit einem Ölreservoir verbundenen, so dass bei Betreiben der Druckluftpistole Öl aus dem Reservoir angesaugt wird, zwangsweise zerstäubt wird und ein so entstehendes Luft-Öl-Gemisch der Haltevorrichtung 1 und den chirurgischen Handstücken 2 zugeführt wird.

In dem bevorzugten Ausführungsbeispiel lagert das Öl in einem Ölreservoir und das Luft-Öl-Gemisch wird mittels der Druckluftpistole in die Haltevorrichtung 1 eingespritzt. Alternativ kann auch ein Ölspray zur Pflege bzw. Ölung verwendet werden. Dabei wird ein Einspritzabschnitt des Ölsprays direkt an dem Anschlussabschnitt 5 der Haltevorrichtung 1 angekoppelt, so dass das sich beim Einspritzen des Öls ausbildende Luft-Öl-Gemisch der Haltevorrichtung 1 zugeführt wird.

Im Anschluss an die Pflege bzw. Ölung werden die chirurgischen Handstücke 2 in einem herkömmlichen Sterilisiergerät sterilisiert. In dem bevorzugten Ausführungsbeispiel ist die Haltevorrichtung 1 so ausgestaltet, dass diese während der Sterilisation in einem Siebkorb platziert werden kann und nach der Sterilisation in dem Siebkorb gelagert werden kann, was die Handhabe verbessert.

### Modifikation des bevorzugten Ausführungsbeispiels

In dem erfindungsgemäßen Ausführungsbeispiel ist der Anschlussabschnitt 5 eingerichtet, um mit einer Filtereinheit, einer Druckluft-/Wasserpistole und einem Ölspray oder einem Ölreservoir koppelbar zu sein. Wie in Fig. 2 gezeigt, ist bei einer Modifikation des bevorzugten Ausführungsbeispiels an dem dem Anschlussabschnitt zugewandten Endabschnitt der zentralen Zuleitung 3 ein Düsenabschnitt 9 ausgebildet. Dieser Düsenabschnitt 9 ist dabei in der Form einer Venturi-Düse ausgestaltet so dass sich ein Querschnitt in Richtung des Fluidstroms zunehmend verringert. An einer Stelle mit relativ kleinem Querschnitt ist zusätzlich abzweigend ein Stechdorn 10 ausgebildet, auf welchen für die Ölung ein Einweg-Ölvorratsbehältnis gesteckt werden kann und welcher eine nachgiebige Dichtung des Einweg-Ölvorratsbehältnisses durchsticht und eine Fluidverbindung zwischen dem Behältnis und dem Düsenabschnitt 9 der zentralen Zuleitung 3 herstellt.

Für die Ölung kann nun an dem Anschlussabschnitt 5 die Druckluftpistole angekoppelt werden, so dass sich bei Betreiben der Druckluftpistole in dem Düsenabschnitt 9 ein Unterdruck ausbildet. Dadurch wird das Öl in den Düsenabschnitt 9 gesaugt, wo es daraufhin zwangsweise zerstäubt wird, so dass sich ein Luft-Öl-Gemisch bildet, welches zur Ölung der Haltevorrichtung 1 und der chirurgischen Handstücke 2 dient. Die Ölung erfolgt dabei solange, bis das Einweg-Ölvorratsbehältnis entleert ist.

Wenn die Haltevorrichtung 1 gemäß der Modifikation des bevorzugten Ausführungsbeispiels den Düsenabschnitt 9 aufweist, muss nach der vorstehend beschriebenen maschinellen Reinigung und Desinfektion die möglicherweise in der Haltevorrichtung 1 und den Handstücken 2 zurückbleibende Flüssigkeit nicht manuell durch die Druckluftpistole ausgeblasen werden. Das Ausblasen erfolgt dabei integral bei der Ölung der Haltevorrichtung 1 bzw. der Handstücke 2.

## Patentansprüche

1. Haltevorrichtung (1) zum Halten und Lagern von zumindest einem medizinischen Instrument, insbesondere einem chirurgischen Handstück (2), in einem Aufbereitungs- und Reinigungsprozess aufweisend
eine zentrale Zuleitung (3),
zumindest einen mit einem Endabschnitt der zentralen Zuleitung (3) verbundenen Leitungsabschnitt (6), an dessen freiem Ende eine Koppeleinrichtung (7) für ein Ankoppeln des zumindest einen medizinischen Instruments angeordnet ist, und
einen an einem anderen Endabschnitt der zentralen Zuleitung (3) ausgebildeten oder angeordneten Anschlussabschnitt (5), der mit einer Vorreinigungseinheit, insbesondere einer Wasser-/Druckluftpistole, und einer Reinigungseinheit, insbesondere eine mit einer Spülleitung verbundenen Filtereinheit, koppelbar ist, **dadurch gekennzeichnet, dass**
der andere Endabschnitt der zentralen Zuleitung (3) in der Art einer Venturi-Düse ausgestaltet ist, an deren Stelle mit relativ kleinem Querschnitt abzweigend ein Ölanschluss als ein mit einem Ölreservoir (11), insbesondere einem Einweg-Ölvorratsbehältnis, koppelbarer Stechdorn (10) ausgebildet ist, wobei
in dem zumindest einen Leitungsabschnitt (6) eine Ventileinrichtung (8) angeordnet ist, welche in einer Grundstellung, in welcher der Leitungsabschnitt (6) und das medizinische Instrument entkoppelt sind, geschlossen ist und sich bei Kopplung des medizinischen Instruments an den Leitungsabschnitt (6) öffnet.

2. Haltevorrichtung (1) nach Anspruch 1, **dadurch gekennzeichnet, dass**
der Anschlussabschnitt (5) als Luer-Lock-Verbindung ausgestaltet ist.

3. Haltevorrichtung (1) nach einem der Ansprüche 1 oder 2, **dadurch gekennzeichnet, dass**
der Anschlussabschnitt (5) mit einer Öleinheit, insbesondere einem Ölspray oder einem Ölreservoir, koppelbar ist.

4. Aufbereitungs- und Reinigungssystem mit
einer Haltevorrichtung (1) nach einem der vorstehenden Ansprüche 1 bis 3,
einer Vorreinigungseinheit, die dafür ausgebildet ist, an den Anschlussabschnitt (5) der Haltevorrichtung (1) angeschlossen zu werden, und
einem Ölreservoir (11), das dafür ausgebildet ist, an den Ölanschluss der Haltevorrichtung (1) angeschlossen zu werden.

## Claims

1. A holding device (1) for holding and storing at least one medical instrument, in particular a surgical handpiece (2), during a processing and cleaning process, the holding device comprising:
a central feedline (3);
at least one line portion (6) connected to an end portion of the central feedline (3), at a free end of which a coupling device (7) is arranged for coupling to the at least one medical instrument; and
a connector portion (5) formed or arranged at another end portion of the central feedline (3), the connector portion being couplable to a pre-cleaning unit, in particular a water gun or a compressed-air gun, and a cleaning unit, especially a filter unit connected to a flushing line, **characterized in that**
the other end portion of the central feedline (3) is configured in the manner of a Venturi nozzle, at the point of which with a comparatively small cross-section an oil connector is formed in a branching manner as a puncture spike (10) that can be coupled to an oil reservoir (11), in particular a disposable oil reservoir, wherein
in the at least one line portion (6) a valve device (8) is arranged, which is closed in a basic position, in which the line portion (6) and the medical instrument are uncoupled, and which opens when the medical instrument is coupled to the line portion (6).

2. The holding device (1) according to claim 1, **characterized in that**
the connector portion (5) is configured as a Luer lock connection.

3. The holding device (1) according to any of claims 1 or 2 **characterized in that**
the connector portion (5) is couplable to an oil unit, in particular to an oil spray or an oil reservoir.

4. A processing and cleaning system, comprising
a holding device (1) according to any of preceding claims 1 to 3,
a pre-cleaning unit configured to be connected to the connector portion (5) of the holding device (1), and
an oil reservoir (11) configured to be connected to the oil connection of the holding device (1).

## Revendications

1. Dispositif de maintien (1) pour le maintien et le stockage d'au moins un instrument médical, en particulier une pièce à main chirurgicale (2), dans un processus de préparation et de nettoyage présentant
une conduite d'alimentation (3) centrale,
au moins une section de conduite (6) reliée à une section d'extrémité de la conduite d'alimentation (3) centrale, à l'extrémité libre de laquelle un dispositif de couplage (7) est disposé pour un couplage de l'au moins un instrument médical, et
une section de raccordement (5) disposée ou formée au niveau d'une autre section d'extrémité de la conduite d'alimentation (3) centrale, section qui peut être couplée à une unité de prénettoyage, en particulier un pistolet à eau/à air comprimé, et à une unité de nettoyage, en particulier une unité de filtrage reliée à une conduite de rinçage, **caractérisé en ce que**
l'autre section d'extrémité de la conduite d'alimentation (3) centrale est configurée comme une buse Venturi, à la place de laquelle un raccordement d'huile est formé bifurquant avec une section transversale relativement petite comme une épine de perçage (10) pouvant être couplée à un réservoir d'huile (11), en particulier un récipient de réserve d'huile à usage unique, dans lequel
un appareil de soupape (8) est disposé dans la au moins une section de conduite (6), appareil qui est fermé dans une position de base dans laquelle la section de conduite (6) et l'instrument médical sont découplés et s'ouvre lors du couplage de l'instrument médical au niveau de la section de conduite (6).

2. Dispositif de maintien (1) selon la revendication 1, **caractérisé en ce que**
la section de raccordement (5) est configurée comme liaison Luer-Lock.

3. Dispositif de maintien (1) selon l'une quelconque des revendications 1 ou 2, **caractérisé en ce que**
la section de raccordement (5) peut être couplée à une unité d'huile, en particulier un spray d'huile ou un réservoir d'huile.

4. Système de préparation et de nettoyage avec
un dispositif de maintien (1) selon l'une quelconque des revendications précédentes 1 à 3,
une unité de prénettoyage qui est formée afin d'être raccordée à la section de raccordement (5) du dispositif de maintien (1) et
un réservoir d'huile (11) qui est formé afin d'être raccordé au raccordement d'huile du dispositif de maintien (1).
